(19) Europäisches Patentamt
European Patent Office
Office européen des brevets

(11) **EP 4 389 186 A1**

(12) **EUROPÄISCHE PATENTANMELDUNG**

(43) Veröffentlichungstag:
**26.06.2024 Patentblatt 2024/26**

(21) Anmeldenummer: **22214795.1**

(22) Anmeldetag: **20.12.2022**

(51) Internationale Patentklassifikation (IPC):
**A61M 25/10** (2013.01)

(52) Gemeinsame Patentklassifikation (CPC):
**A61M 25/10184; A61B 5/02007; A61B 5/6853;**
A61M 25/10187; A61M 25/104; A61M 2025/1052

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(71) Anmelder: **BIOTRONIK AG**
**8180 Bülach (CH)**

(72) Erfinder:
• **Quint, Bodo**
**79802 Dettighofen (CH)**
• **Risch, Fabian**
**8239 Doerflingen (CH)**

(74) Vertreter: **Biotronik Corporate Services SE**
**Corporate Intellectual Property**
**Sieversufer 7-9**
**12359 Berlin (DE)**

(54) **DIE DYNAMISCHE ABLEITUNG DES INFLATIONSVERHALTENS EINES DILATATIONSKATHETERS**

(57) Die Erfindung betrifft ein Ballonkathetersystem (1), mit einem Katheter (2), der einen Katheterschaft (20) aufweist, der ein Lumen (21) umgibt, wobei der Katheter (2) weiterhin einen Ballon (22) aufweist, der mit einem distalen Ende (20a) des Katheterschafts (20) verbunden ist, so dass zum Inflatieren des Ballons (22) ein fluides Inflationsmedium (3) in einen Balloninnenraum (23) des Ballons (22) über das Lumen (21) einleitbar ist oder zum Deflatieren des Ballons (22) aus dem Balloninnenraum (23) abziehbar ist. Erfindungsgemäß ist vorgesehen, dass das Ballonkathetersystem (1) einen Drucksensor (4) aufweist, der dazu ausgebildet ist, einen momentanen Druck (p) des Inflationsmediums (3) im Lumen (21) beim Inflatieren oder Deflatieren des Ballons (22) zu messen, sowie einen Flusssensor (5), der dazu ausgebildet ist, einen momentanen Volumenfluss (v) des Inflationsmediums (3) beim Inflatieren oder Deflatieren des Ballons (22) zu messen, und wobei das Ballonkathetersystem (1) eine Auswertungseinheit (6) aufweist, die dazu konfiguriert ist, den momentanen Druck und den momentanen Volumenfluss beim Inflatieren oder Deflatieren des Ballons (22) aufzuzeichnen und hieraus einen zeitlichen Verlauf des Strömungswiderstands (R) zu ermitteln.

FIG. 10

EP 4 389 186 A1

**Beschreibung**

Die Erfindung betrifft ein Ballonkathetersystem sowie ein Verfahren zur Analyse des Ballonverhaltens.

Liegt der Ballon eines Ballonkatheters bei einer Angioplastie im Bereich der zu beseitigenden Stenose, wird der Ballon in der Regel unter hohem Druck (bis zu 16 atm) mit einem fluiden Inflationsmedium, das in den Balloninnenraum eingeleitet wird, inflatiert, wobei sich der Ballon insbesondere in der radialen Richtung (d.h. senkrecht zur axialen Richtung des Ballonkatheters) ausdehnt und der Durchmesser des Ballons in der radialen Richtung entsprechend zunimmt. Durch den starken Druck, den der Ballon auf die Gefäßwand ausübt, werden z.B. bei einer Angioplastie die Kalkablagerungen in die Gefäßwand gedrückt, und der Gefäßdurchmesser vergrößert sich. Bei einem derartigen Eingriff ist es natürlich wünschenswert, dass der behandelnde Arzt den beim Inflatieren des Ballons sowie insbesondere später beim Deflatieren des Ballons Rückmeldungen über das Ballonveralten beim Inflatieren und Deflatieren erhält, so dass die Angioplastie möglichst präzise und kontrolliert durchgeführt werden kann. Grundsätzlich ist hierbei für den behandelnden Arzt von Interesse, wann der Ballon beim Inflatieren in Kontakt mit der Gefäßwand gerät, wieviel Energie für das Aufbrechen bzw. die Dilation der Stenose notwendig war sowie wann der Ballon bei der Deflation den Gefäßkontakt wieder verliert.

Die WO 2014/210450 offenbart Systeme und Verfahren zum Messen und Bereitstellen einer Echtzeitanzeige von Innendurchmesser, Gegenkraft und Compliance einer biologischen Testleitung, die eine Läsion umfassen kann. Bei diesem Verfahren wird eine Referenz-Volumen-Druck-Charakteristik ermittelt, die durch Inflatieren eines Referenzballons mit einem Satz bekannter physikalischer Eigenschaften bei einer bekannten und festen Inflationsrate auf ein bekanntes und festes Volumen erzeugt wird, wobei die mit inkrementalen Volumenänderungen verbundenen Drücke gemessen werden. Weiterhin wird der Außendurchmesser des Referenzballons während des Inflatierens und der Expansion gemessen und der Außendurchmesser des expandierenden Referenzballons wird auf die inkrementellen Volumenänderungen und die zugehörigen Drücke abgebildet. Ferner wird mindestens eine Referenz-Volumen-Druck-Charakteristik ermittelt, die durch Inflatieren eines Testballons erzeugt wird, wobei der Testballon den Satz bekannter physikalischer Eigenschaften des Referenzballons aufweist, bei der bekannten und festen Aufblasrate und dem festen Volumen innerhalb der biologischen Prüfleitung (Körpergefäß mit vergleichbaren Gefäßlumen an einer Stelle ohne Stenose), und wobei der mit den inkrementellen Volumenänderungen verbundene Druck gemessen wird. Sodann wird die mindestens eine Referenz-Volumen-Druck-Charakteristik mit der mindestens einen Test-Volumen-Druck-Charakteristik verglichen und mindestens ein Innendurchmesser der biologischen Testleitung, mindestens eine Gegenkraft der biologischen Testleitung auf den expandierenden Testballon und mindestens einer Messung einer Volumen-Druck-Charakteristik der biologischen Leitung und/oder Läsion werden bestimmt bzw. durchgeführt.

Die Volumenerfassung bei Ballonkathetersystemen ist regelmäßig mit mehreren Fehlerquellen behaftet. So kann z.B. bei einer händischen Bedienung einer Inflationspumpe die Restmenge an Luft, die sich im Inflationssystem befindet, eine Störgröße darstellen. Diese Störgrößen, wie der Einschluss von Luft im Katheter, kann Auswertungen der oben beschrieben Art erheblich erschweren.

Weiterhin ist eine Volumenerfassung, die ausschließlich die motorische Füllung des Katheters über einen Kolben erfasst insofern problematisch, da sie von dem Entlüftungszustand des druckerzeugenden Systems abhängig ist.

Weiterhin ist die Aufnahme einer Test-Volumen-Druck-Charakteristik in einer biologischen Prüfleitung (Körpergefäß mit vergleichbarem Gefäßlumen an einer Stelle ohne Stenose) zumindest mit einem zusätzlichen Aufwand verbunden. Dieser zusätzliche Aufwand verlängert die Prozedur und führt zu einer höheren Belastung des zu behandelnden Patienten. Zusätzlich kann nicht sichergestellt werden, dass für jeden Patienten eine geeignete biologische Prüfleitung zur Verfügung steht. Ferner ist die Aufnahme einer Test-Volumen-Druck-Charakteristik nur mit einem Ballonkathetersystem ohne Stent möglich.

Der vorliegenden Erfindung liegt hiervon ausgehend die Aufgabe zugrunde, ein Ballonkathetersystem sowie ein Verfahren anzugeben, wobei das Ballonkathetersystem bzw. das Verfahren es ermöglichen sollen ein Ballonverhalten beim Inflatieren und/oder Deflatieren zu ermitteln, ohne Referenzmessungen des Katheters zu benötigen. Vorzugsweise soll die Erfindung weiterhin das Vorliegen von Restluft im Katheter verhindern, um eine Störung der Messergebnisse zu vermeiden.

Diese Aufgabe wird durch ein Ballonkathetersystem mit den Merkmalen des Anspruchs 1 sowie durch ein Verfahren mit den Merkmalen des Anspruchs 14 gelöst. Vorteilhafte Ausgestaltungen dieser Erfindungsaspekte sind in den entsprechenden Unteransprüchen angegeben und werden nachfolgend beschrieben.

Gemäß Anspruch 1 wird ein Ballonkathetersystem, insbesondere für die Angioplastie, offenbart, mit:

- einem Katheter, der einen Katheterschaft aufweist, der ein Lumen umgibt, wobei der Katheter weiterhin einen Ballon aufweist, der mit einem distalen Ende des Katheterschafts verbunden ist, so dass zum Inflatieren des Ballons ein fluides Inflationsmedium in einen Balloninnenraum des Ballons über das Lumen einleitbar ist oder zum Deflatieren des Ballons aus dem Balloninnenraum abziehbar ist.

Erfindungsgemäß ist vorgesehen, dass das Ballonkathetersystem einen Drucksensor aufweist, der dazu ausgebildet ist, einen momentanen Druck des Inflationsmediums im Lumen oder im Balloninnenraum beim Inflatieren oder Deflatieren des Ballons zu messen, sowie einen Flusssensor, der dazu ausgebildet ist, einen Volumenfluss des Inflationsmediums beim Inflatieren oder Deflatieren des Ballons zu messen, und wobei das Ballonkathetersystem eine Auswertungseinheit aufweist, die dazu konfiguriert ist, den momentanen Druck und den momentanen Volumenfluss beim Inflatieren oder Deflatieren des Ballons zu speichern und aus den gemessenen Werten des momentanen Drucks und des momentanen Volumenflusses einen zeitlichen Verlauf des Strömungswiderstands zu ermitteln. Der Strömungswiderstand wird aus dem Verhältnis von momentanem Druck zu momentanem Volumenfluss, also Volumen pro Zeit, ermittelt.

Durch die systematisierte Kenntnis eines Ballonkathetersystems lassen sich ohne detaillierte Kenntnis der Inflations-Deflationscharakteristik zusätzliche Informationen für den klinischen Einsatz gewinnen. Die Detailierungstiefe der Aussagen kann über systematische Erkenntnisse zu einem Katheterdesign verfeinert werden. Die hierzu benötigte sensorische Datenerfassung bedarf keiner hohen Messtechnischen Präzision da eine detaillierte Diskussion der Datenlage erlaubt, Messwerte retrospektiv (deduktiv) zu normieren, um systematische Datenerfassungsfehler zu kompensieren.

Eine Kurvendiskussion der dynamischen Datenerfassung z. B. am Luer eines Katheteranschlusses, insbesondere Druck und Volumenfluss des fluiden Inflationsmediums vermag ggf. mit systematischen Hintergrundinformationen eines Katheterdesigns Folgendes in vorteilhafterweise zu leisten:

- die Ermittlung einer unvollständigen Katheterdilatation und die Empfehlung einer Nachdilatation,
- die Ermittlung des dynamischen Gegendruckes über das Gewebe des Dilatations- oder Implantationsortes,
- die Ermittlung einer Arbeit, die für ein dilatiertes Volumen angefallen ist und somit die Möglichkeit eine Stenose zu klassifizieren und die Art und Beschaffenheit der Stenose besser abzuschätzen,
- die Schätzung der Restluftmenge im Katheter,
- die Kompensation fertigungsbedingter Schwankungen des Strömungswiderstandes im Katheterschaft des Katheters,
- die Berücksichtigung viskoelastischer Deformation des Ballons und somit präzisere Durchmesserangaben als sie durch ein Compliance-Chart ermöglicht sind,
- eine Klassifizierung der Kinetik der plastischen Gewebeveränderung und somit optimierte klinische Interventionen,
- für ballonbasierte Implantationssysteme kann indiziert werden, ab wann ein Stent abgesetzt wurde und folgend kann eine Umschaltung der Datenaufbereitung für eine eventuelle Nachdilatation des Stents durch den Ballon erfolgen,
- Feststellung von Defiziten bei der Dilation (beispielsweise ein deutlich limitiertes Volumen des Dilatationsvorgangs) und eine auf der Feststellung basierend Warnung und Empfehlung über eine Nachdilatation,
- die Zeit, ab welcher ein spezifizierter Druck - übertragen von einem Inflationssystem - physikalisch auch am Ballon anliegt, ist dem Nutzer unbekannt und wird nun detektierbar und auch visualisierbar.

Die erfindungsgemäße Lösung ermöglicht die Detektion unerwünschter und vom erwarteten Verlauf abweichende Effekte bei der Dilation einer Stenose. Hierdurch können etwaige Komplikationen bei der Dilation einer Stenose vermieden und der Prozess der Dilation der Stenose verbessert werden.

Gemäß einer bevorzugten Ausführungsform der Erfindung ist vorgesehen, dass die Auswertungseinheit dazu konfiguriert ist, mit Hilfe des zeitlichen Verlaufes des Strömungswiderstands zumindest eine Größe der folgenden Größen zu bestimmen:

- einen Zeitpunkt zu dem der Ballon beim Inflatieren eine Gefäßwand des Patienten kontaktiert,
- eine Energie, die für eine Dilation einer Stenose des Patienten notwendig war,
- einen Zeitpunkt zu dem die Stenose dilatiert ist.

Der Strömungswiderstand, der dem Verhältnis von einem an ein Ballonkathetersystem angelegten Druck zu einem Volumenfluss in das Ballonkathetersystem entspricht, kann als ein Maß für den Widerstand betrachtet werden, gegen den der Ballon inflatiert werden muss. Für den Fall, dass der Ballon ohne eine äußere auf den Ballon einwirkende Kraft inflatiert wird, wird der Strömungswiderstand durch ballonspezifische Eigenschaften bestimmt, wie beispielsweise durch die Ballongeometrie, die Ballondimensionen, die Faltung des Ballons, dem Material der Ballonwand und der Stärke der Ballonwand. Für den Fall, dass der Ballon an einer Gefäßwand anliegend inflatiert wird und somit das Gefäß beim Inflatieren aufdehnt, wird der Strömungswiderstand ebenfalls durch das Gefäß bestimmt, welches der Balloninflation entgegenwirkt. Wird der Ballon beispielsweise innerhalb einer Stenose inflatiert, sodass es zu einer Weitung der Stenose kommt, hängt der Strömungswidersand beispielsweise auch von der Beschaffenheit der Stenose, der Dimensionen der Stenose oder dem Grad der Kalzifikation der Stenose ab.

Ab dem Zeitpunkt zu dem der Ballon beim Inflatieren eine Gefäßwand des Patienten kontaktiert wird der Strömungswiderstand verglichen mit demjenigen eines Ballons ohne äußere Krafteinwirkung auf den sich inflatierenden Ballon (beispielsweise bei der Inflation des Ballons außerhalb eines Gefäßes an der Luft) bei entsprechendem Volumenstrom in den Ballon größer sein und stärker ansteigen. Bei der Dilation einer Stenose weist der Strömungswiderstand abhängig von der Zeit, bei einem Volumenstrom in den Ballon, ein Maximum auf und fällt danach wieder mit zunehmender Inflation des Ballons auf den Strömungswiderstand eines Ballons ohne äußere Krafteinwirkung auf den sich inflatierenden Ballon ab. Ab diesem Zeitpunkt dominiert der Strömungswiderstand, der durch die Dehnung des Ballons selbst entsteht, und Strömungswiderstände, die aus der Dehnung der Stenose bzw. des Gefäßes resultieren, sind demgegenüber verschwindend gering. Dieser Zeitpunkt kann als Zeitpunkt verstanden werden, an dem die Stenose dilatiert ist.

Bevorzugt ist der Zeitpunkt zu dem die Stenose dilatiert ist, derjenige Zeitpunkt zu dem der zeitliche Verlauf des Strömungswiderstandes nach Erreichen eines Maximums des Strömungswiderstandes eine Abweichung von dem zeitlichen Verlauf des Strömungswiderstandes ohne äußere Krafteinwirkung auf den Ballon bei entsprechenden Volumenstrom in den Ballon von 5 % oder weniger, weiterhin bevorzugt von 1 % oder weniger und besonders bevorzugt von 0,1 % oder weniger erreicht.

Die Energie $W_{Stenose}$, die für die Dilation der Stenose des Patienten notwendig war, entspricht der (Volumen-)Arbeit $W_{gesamt}$, die zur Inflation des Ballons zwischen dem Zeitpunkt ti zu dem der Ballon beim Inflatieren eine Gefäßwand des Patienten kontaktiert und dem Zeitpunkt $t_2$ zu dem die Stenose dilatiert ist verrichtet wurde, abzüglich des Anteils der verrichteten Arbeit $W_{Ballon}$, die ausschließlich zur Dehnung des Ballons selbst im Zeitraum zwischen dem Zeitpunkt ti und dem Zeitpunkt $t_2$ verwendet wurde ($W_{Ballon}$ entspricht der Arbeit, die bei der Inflation des Ballons ohne äußere Krafteinwirkung auf den sich inflatierenden Ballon verrichtet würde).

Für die Volumenarbeit W gilt:

$$W = \int pV dp dV$$

mit Druck p und Volumen V.

Für die Leistung P gilt:

$$P = \frac{p^2}{\frac{dV}{dt}} = \frac{p^2}{v}$$

mit Volumenstrom v.

Daraus folgt für $W_{gesamt}$:

$$W_{gesamt} = \int_{t_1}^{t_2} \frac{p^2(t)}{v(t)}\, t\, dt$$

Mit dem Strömungswiderstand $R = \frac{p}{v}$ ergibt sich:

$$W_{gesamt} = \int_{t_1}^{t_2} p(t) R(t)\, dt$$

Der Strömungswiderstand R(t) setzt sich hierbei aus einem Strömungswiderstand $R_{Stenose}$ resultierend aus der Dilation der Stenose und aus einem Strömungswiderstand $R_{Ballon}$ resultierend aus der Dehnung des Ballons zusammen. Damit kann die Energie $W_{Stenose}$, die für die Dilation der Stenose des Patienten notwendig war, wie folgt berechnet werden:

$$W_{Stenose} = \int_{t_1}^{t_2} p(t)(R(t) - R_{Ballon}(t))\, dt$$

Der zeitliche Verlauf des Strömungswiderstands R wird von der Auswertungseinheit ermittelt.

Ferner ist die Auswertungseinheit dazu konfiguriert den zeitlichen Verlauf des Strömungswiderstands $R_{Ballon}$ zwischen den Zeitpunkten $t_1$ und $t_2$ zu interpolieren. Bevorzugt ist die Auswertungseinheit dazu konfiguriert den zeitlichen Verlauf des Strömungswiderstands $R_{Ballon}$ zwischen den Zeitpunkten $t_1$ und $t_2$ wie folgt zu interpolieren:

$$R_{Ballon}(t) = \frac{(R_{Ballon}(t_2) - R_{Ballon}(t_1)) * (t - t_1)}{t_2 - t_1} + R_{Ballon}(t_1)$$

Eine Bestimmung der Energie $W_{Stenose}$, die für die Dilation der Stenose des Patienten notwendig war, kann somit insbesondere analytisch, numerisch oder durch Summierung der Produkte $p(t)(R(t) - R_{Ballon}(t))$ für die einzelnen Messwerte in dem Zeitintervall $[t_1; t_2]$ erfolgen.

Als diagnostisches Ergebnis erhält der Arzt aus der zur Dilation notwendigen Energie insbesondere Informationen zu einer Härte oder mechanischen Festigkeit der Stenose.

Weiterhin ist gemäß einer Ausführungsform der Erfindung vorgesehen, dass der Katheter einen mit dem Lumen in Strömungsverbindung stehenden Katheteranschluss (z. B. an einem proximalen Ende des Katheterschafts) aufweist, wobei der Drucksensor dazu ausgebildet ist, den momentanen Druck am Katheteranschluss zu messen und/oder wobei der Flusssensor dazu ausgebildet ist, den Volumenfluss am Katheteranschluss zu messen. Insbesondere kann der Katheteranschluss als Luer-Verbinder ausgebildet sein.

Alternativ oder ergänzend zu dem Flusssensor, ist die Auswertungseinheit dazu ausgebildet von einer Vorrichtung Daten zu empfangen, wobei die Daten den momentanen Druck des Inflationsmediums im Lumen oder im Balloninnenraum beim Inflatieren oder Deflatieren des Ballons sowie den Volumenfluss des Inflationsmediums beim Inflatieren oder Deflatieren des Ballons umfassen.

Im Rahmen der vorliegenden Erfindung werden Komponenten oder Orte, die entlang des Ballonkatheters näher am Anwender (Arzt) des Ballonkatheters liegen als proximal bezeichnet. Entsprechend werden Komponenten oder Orte, die weiter vom Anwender des Ballonkatheters entfernt sind als distal bezeichnet.

Gemäß einer Ausführungsform der Erfindung kann es sich bei dem Flusssensor um einen Coriolis-Flusssensor handeln. Bei einem solchen Flusssensor wird in bekannter Weise das Coriolis-Prinzip verwendet, um einen Massenfluss bzw. einen Volumenfluss des Inflationsmediums zu messen. Aufgrund der erzeugten Flussraten, wie auch der direkten Masseabhängigkeit erscheint die Volumenerfassung nach dem Coriolis-Prinzip vorteilhaft. Dieses Sensorprinzip ist prinzipiell miniaturisierbar und daher für den Kathetereinsatz geeignet. Aufgrund der Abwesenheit von Fügungen, bzw. Materialübergängen zur Sensorrealisierung erscheint dieser Sensortyp insbesondere auch zur Re-Sterilisation mittels Flüssigkeitssterilisation geeignet. Alternativ können ggf. auch thermische Volumenflusssensoren verwendet werden.

Weiterhin ist gemäß einer Ausführungsform der Erfindung vorgesehen, dass das Ballonkathetersystem eine Pumpe zum Inflatieren und/oder Deflatieren des Ballons mit dem fluiden Inflationsmedium aufweist.

Gemäß einer Ausführungsform ist der Drucksensor in die Pumpe integriert.

Hierbei kann weiterhin der Flusssensor des Ballonkathetersystems gemäß einer Ausführungsform der Erfindung in eine Sensoreinheit integriert sein, die dazu ausgebildet ist, die Pumpe mit dem Katheteranschluss zu verbinden, d.h., eine Strömungsverbindung zwischen der Pumpe und dem Katheteranschluss herzustellen, über die das Inflationsmedium in den Balloninnenraum eingeleitet werden kann (bzw. aus dem Balloninnenraum abgezogen werden kann).

Gemäß einer alternativen Ausführungsform der Erfindung ist vorgesehen, dass der Drucksensor und der Flusssensor in eine Sensoreinheit integriert sind, die wiederum dazu ausgebildet ist, den Katheteranschluss und die Pumpe miteinander zu verbinden, d.h., eine Strömungsverbindung zwischen der Pumpe und dem Katheteranschluss herzustellen, über die das Inflationsmedium in den Balloninnenraum eingeleitet werden kann (bzw. aus dem Balloninnenraum abgezogen werden kann).

Hierbei ist gemäß einer Ausführungsform weiterhin vorgesehen, dass die Sensoreinheit dazu ausgebildet ist, die gemessenen momentanen Druckwerte und die gemessenen momentanen Volumenflusswerte drahtlos an die Auswertungseinheit zu übermitteln.

Gemäß einer Ausführungsform der Sensoreinheit ist vorgesehen, dass die Sensoreinheit einen Energiespeicher (z.B. eine Batterie) aufweist. Weiterhin weist die Sensoreinheit vorzugsweise eine Spule zur Herstellung der drahtlosen Kommunikation auf (z.B. Funkverbindung, insbesondere Nahfeldkommunikation) sowie insbesondere zum Laden des Energiespeichers auf.

Weiterhin kann die Sensoreinheit eine Antenne (z.B. bei einer Übertragungsfrequenz von 433 MHz) zum Senden und Empfangen von Funksignalen aufweisen.

Weiterhin ist gemäß einer Ausführungsform der Erfindung vorgesehen, dass das Ballonkathetersystem eine Datenerfassungseinheit aufweist, die zur Eingabe einer oder mehrerer den Katheter charakterisierenden Parameter konfiguriert ist (beispielsweise einer Produktnummer, die beispielsweise durch einen Barcode dargestellt wird), um insbesondere eine Identifikation des verwendeten Katheters zu ermöglichen.

Weiterhin ist gemäß einer Ausführungsform der Erfindung vorgesehen, dass die Auswertungseinheit dazu konfiguriert ist, den jeweiligen Parameter bei der Bestimmung der mindestens einen Größe (siehe oben) zu verwenden. Die Auswertungseinheit kann hierbei optional die Parameter bzw. katheterspezifischen Zusatzinformationen verwenden, um die Signalauswertung und Datenaufbereitung zu verbessern/präzisieren.

Gemäß einer Ausführungsform der Erfindung kann die Auswertungseinheit auch in die Datenerfassungseinheit integriert sein oder geeignet mit dieser kommunizieren. Diese beiden Komponenten können insbesondere ein einheitliches Gerät bilden. Ein solches Gerät kann z.B. durch einen Computer gebildet sein, auf dem eine entsprechende Software ausgeführt wird.

Weiterhin ist gemäß einer Ausführungsform der Erfindung vorgesehen, dass der Katheteranschluss ein Eingangslumen aufweist, das mit dem Lumen des Katheterschafts in Strömungsverbindung steht. Hierbei weist die Sensoreinheit einen Auslassbereich zum Auslassen des Inflationsmediums aus der Sensoreinheit auf, wobei der Auslassbereich dazu ausgebildet ist, formschlüssig in das Eingangslumen einzugreifen. Hierdurch kann ein Restvolumen des Katheteranschlusses mit Vorteil vermieden werden. In einen solchem Restvolumen könnte sich andernfalls Luft sammeln, die die Messergebnisse aufgrund der Kompressibilität der Luft verfälschen könnte.

Vorteilhafterweise ist das Ballonkathetersystem dazu ausgebildet, einen Druckabfall über das Eingangslumen zu bestimmen. Hierbei wird der Druck in Richtung des Volumenflusses vor dem Eingangslumen und im Eingangslumen bestimmt. Aus der Differenz dieser beiden Druckwerte kann der Volumenstrom bestimmt werden.

Gemäß einer weiteren Ausführungsform ist der Katheterschaft Teil des Flusssensor. Hierdurch kann eine präzise Bestimmung des Volumenflusses durch den Katheterschaft erzielt werden.

Alternativ zu dem wie vorliegend ausgestalteten Katheteranschluss/Auslassbereich kann das Restvolumen im Katheteranschluss auch rechnerisch korrigiert werden (z.B. durch die Auswertungseinheit), wenn das besagte Restvolumen der Luft im Katheter durch entsprechende Vorcharakterisierung bekannt ist.

Ein weiterer Aspekt der vorliegenden Erfindung betrifft ein Verfahren zum Inflatieren eines Ballons eines Katheters mit einem fluiden Inflationsmedium, aufweisend die Schritte:

- Messen und Aufzeichnen eines momentanen Volumenflusses des Inflationsmediums und eines momentanen Drucks des Inflationsmediums beim Inflatieren oder Deflatieren des Ballons, und
- automatisches Bestimmen eines zeitlichen Verlaufs des Strömungswiderstands beim Inflatieren oder Deflatieren des Ballons mittels gemessener [oder aufgezeichneter] Werte des momentanen Drucks und des momentanen Volumenflusses.

Gemäß einer Ausführungsform des Verfahrens ist vorgesehen, dass mit Hilfe des Strömungswiderstands bzw. des zeitlichen Verlaufs des Strömungswiderstands zumindest eine Größe der folgenden Größen automatisch bestimmt wird:

- ein Zeitpunkt, zu dem der Ballon beim Inflatieren eine Gefäßwand des Patienten kontaktiert,
- eine Energie, die für eine Dilation einer Stenose des Patienten notwendig war,
- ein Zeitpunkt, zu dem der Ballon beim Deflatieren einen Gefäßkontakt wieder verliert.

Weiterhin ist gemäß einer Ausführungsform des Verfahrens vorgesehen, dass die Auswertungseinheit dazu konfiguriert ist, zum Bestimmen der jeweiligen Größe den Strömungswiderstand automatisch zu integrieren.

Im Folgenden sollen Ausführungsformen der Erfindung sowie weitere Merkmale und Vorteile der Erfindung anhand der Figur erläutert werden. Es zeigen:

Fig. 1 eine Informationsreduktion auf die Signale Volumen(fluss), und Druck - (das ermittelte integrale Flusssignal oder gemessene Volumensignal wird nun als Abszisse gewählt). Um prinzipiell die Reproduzierbarkeit solcher Messungen zu zeigen werden drei aufeinander folgende Katheterinflationen im unbelasteten Katheterzustand dargestellt. Es erfolgte eine "langsame Inflation" zu einem Druck von 7 bar;

Fig. 2 aufeinanderfolgende Inflationen eines Katheters in einem begrenzenden Silikonschlauch. Es erfolgen Inflationen zu dem nominalen Maximaldruck des Katheters (16bar). Die erste Kurve zeigt eine schematische Inflation in der Dimension Volumensignal / Druck. Die zweite Kurve zeigt eine schematische Deflation in derselben Dimension;

Fig. 3 - 4    Gegenüberstellung der ersten Katheterinflation gemäß Figur 3 und der dritten Katheterinflation gemäß Figur 4 in dem begrenzenden Silikonschlauch;

Fig. 5    eine Darstellung der Gesamtenergie der Katheterinflatation (z-Achse) entsprechend der geleisteten Volumenarbeit am Luer für eine Stentinflation ohne äußere Restriktion und eine Inflation eines Silikonschlauches.

Fig. 6    den Zusammenhang zwischen Druck und Volumen bei der Inflation eines Ballons in einem Silikonschlauch ohne Draht;

Fig. 7    den Zusammenhang zwischen Druck und Volumen bei der Inflation eines Ballons in einem Silikonschlauch mit im Lumen des Silikonschlauchs angeordnetem Draht zur Volumenbegrenzung des Ballons;

Fig. 8    die Darstellung des Versuches mit dem Silikonschlauch in der Strömungswiderstand-Volumen-Ebene;

Fig. 9    eine schematische Darstellung der Auswertung der gemessenen Druck- und Volumenflusswerte;

Fig. 10    zeigt eine bevorzugte Ausführungsform des erfindungsgemäßen Ballonkathetersystems;

Fig. 11    den Katheteranschluss gemäß Figur 10 und ein Auslassbereich der Sensoreinheit gemäß Figur 10, der zum Eingreifen in den Katheteranschluss ausgebildet ist, um ein Restvolumen des Katheteranschlusses zu vermeiden;

Fig. 12    der Auslassbereich gemäß Fig. 11, wobei dieser betimmungsgemäß in den Katheteranschluss eingeführt ist.

Nachfolgend werden Ausführungsformen und Beispiele der erfindungsgemäßen Lösung näher beschrieben, wobei der der Erfindung zugrundeliegende Algorithmus aufgezeigt werden soll. Hierbei wird dargelegt werden, welche Messgrößen ausgewertet werden, warum auf eine Referenzmessung verzichtet werden kann (ausreichend ist die Kenntnis des Katheterdesigns) und wie mit Störgrößen umgegangen werden kann.

Der Algorithmus wird anhand einer Darstellung der Inflations- und Deflations-Kurven im Raum Druck/Volumen/Strömungswiderstand gezeigt. Der Strömungswiderstand wird über das Verhältnis des angelegten Drucks p am Katheteranschluss 24 und dem sich dort einstellenden Volumenfluss V'=dV/dt errechnet (p/V') und entspricht der Dimension bar/(ml/s) entsprechend der elektrisch analogen Widerstandsdefinition (U/ I).

In den einzelnen Graphen wird eine exponentiell wirkende Skalierung genutzt, um kleine Strömungswiderstandsänderungen besser visualisieren zu können. Der Strömungswiderstand unterliegt einer exponentiellen Transformation, die der Wurzelfunktion entspricht, jedoch einen Vorzeichenwechsel akzeptiert, um auch eine Umkehr der Flussrichtung darstellen zu können.

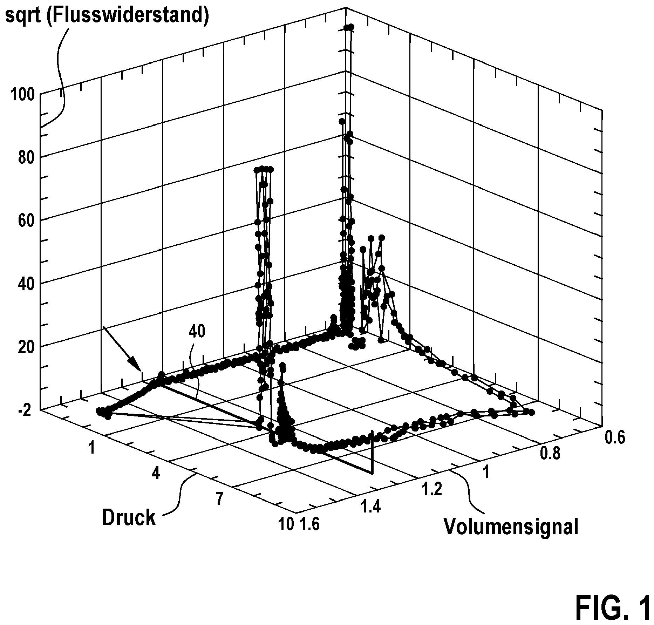

Fig. 1 zeigt Informationsreduktion auf die Signale Volumen(fluss) und Druck (das ermittelte integrale Flusssignal oder gemessene Volumensignal wird als Abszisse gewählt). Um prinzipiell die Reproduzierbarkeit solcher Messungen zu zeigen werden drei aufeinander folgende Balloninflationen im unbelasteten Katheterzustand dargestellt. Es erfolgte eine im Vergleich zu einer typischen Inflation "langsame Inflation" zu einem Druck von 7 bar.

Bei einem realen Kathetereinsatz wird der Gewebekontakt zu einer zeitlich veränderten Inflationskinetik und - auch nach erfolgter Dilatation - reduziertem Volumensignal führen. Interessanter Weise ist das charakteristische Volumen, ab welchem die elastisch im Ballonsegment und in der Restluft gespeicherte Energie des Katheters noch die Deflation unterstützt, sehr gut während der Deflation detektierbar (Pfeil in der Fig. 1). Entsprechend kann das Volumensignal (Linie 40 in der Fig. 1) ermittelt werden, ab welchem auch bei der Inflation der Übergang von "Restluftkompression" zu der geometrischen Begrenzung des Ballons, bzw. des Gewebes erfolgt. Es besteht sogar die Möglichkeit, ohne Kenntnis der Balloncharakteristik auszuwerten, ob und ab welchem Füllgrad entweder der Ballonkontakt oder gar schon der Gewebekontakt begrenzend wirkt. Somit ist das durch den Kathetereinsatz verdrängte Volumen während des Kathetereinsatzes schätzbar. Unter Kenntnis des Katherterdesigns sind Füllvolumina des Katheters und deren druckbedingte Veränderung systematisierbar.

Somit wird es möglich, das Füllverhalten des Katheters mit Design-Vorwissen und der praktischen Echtzeitmessung von Druck und Volumenfluss zu ermitteln. Unter Design-Vorwissen werden dabei charakteristische Volumeneigenschaften und das viskoelastische Verhalten des Ballons unter nicht belasteten Einsatzbedingungen (Inflation des Ballons außerhalb des Gefäßes an Luft) verstanden.

Produktionsbedingte Abweichungen und Viskositätseinflüsse des Röntgenkontrastmittels sind kompensierbar, da diese über den Schaftwiderstand (d.h. dem Strömungswiderstand im Schaft des Katheters) während des Kathetereinsatzes tatsächlich erfassbar sind.

Durch die Visualisierung anhand des Volumensignales wird auch plausibel, warum rationale Erfassungskriterien, wie zum Beispiel "das Anfangsvolumen entspricht dem Endvolumen nach Deflation", genutzt werden können, um gröbere Abweichungen, wie Leckagen, oder unter Ausschluss dieses Fehlers auch eine sensorinduzierte Erfassungsdrift zu kompensieren.

Somit kann eine retrospektive Simulation (d.h. nach erfolgter Deflation) erfasster Daten während des Kathetereinsatzes das Verhalten des unbelasteten Katheters beschreiben und dieses Verhalten ist mit aktuellen Messdaten des Einsatzes des Katheters präzisierbar.

Werden während des Kathetereinsatzes signifikante Abweichungen von diesem systematisierten Katheterverhalten registriert, so sind diese dem Gewebekontakt zuzuschreiben, insbesondere einem Kontakt mit einer den Ballon umgebenden Gefäßwand.

Die nachfolgenden Beispiele bezüglich desselben Kathetertyps sollen aufzeigen, welche Aussagen hierdurch ermöglicht werden.

Der Katheter wird wiederholt in einem begrenzenden elastischen Silikonschlauch aufgeblasen. Silikone weisen relativ schlechte elastische Eigenschaften auf bzw. zeigen ein ausgeprägtes viskoelastisches Verhalten.

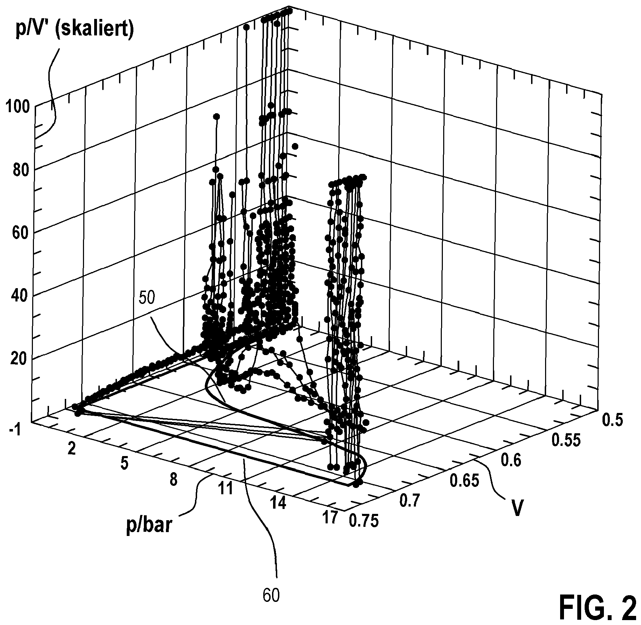

Diesbezüglich zeigt Fig. 2 aufeinanderfolgende Inflationen eines Katheters in dem begrenzenden Silikonschlauch. Hierbei erfolgen Inflationen zu dem nominalen Maximaldruck des Katheters (16 bar), wobei die erste Kurve 50 eine Inflation in der Dimension Volumen / Druck anzeigt; die zweite Kurve 60 entspricht einer Deflation in derselben Dimension. Die Messwerterfassung (Volumensignal und Druck) erfolgt am Katheteranschluss 24, insbesondere am Lueranschlusspunkt des Katheters 2. Der Strömungswiderstand R (z-Achse) unterliegt einer exponentiellen Transformation, die der Wurzelfunktion entspricht, jedoch auch einen Vorzeichenwechsel akzeptiert (siehe oben).

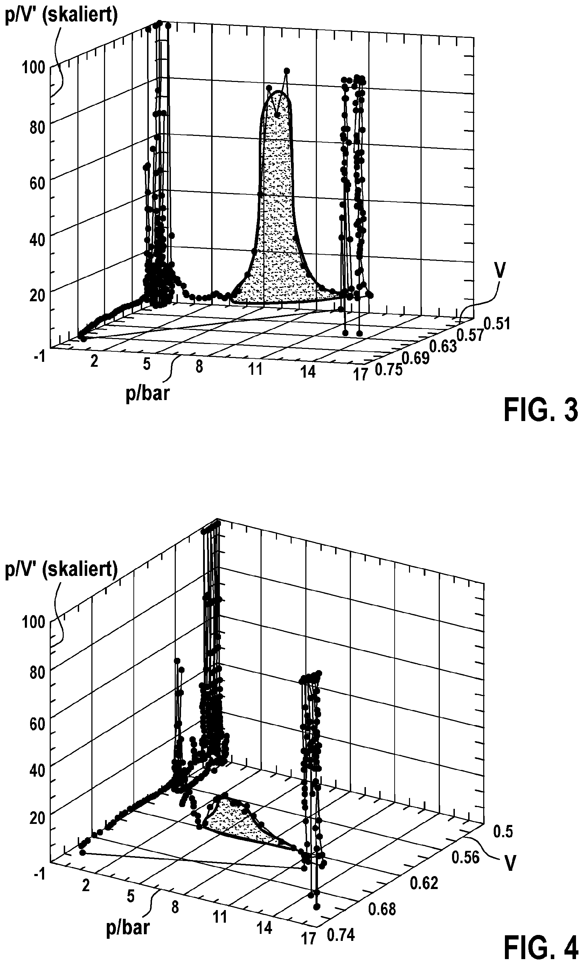

Die Figuren 3 und 4 zeigen eine Gegenüberstellung der ersten Katheterinflation (Kurve über dem schraffierten Bereich in der Figur 3) und der dritten Katheterinflation (Kurve über dem schraffierten Bereich in der Figur 4) in dem begrenzenden Silikonschlauch.

Anhand der Figuren 3 und 4 ist ersichtlich, dass der Strömungswiderstand die nun zusätzlich zu leistende Energie für die Verformung des Silikonschlauches erfasst. Die Verformung des Silikonschlauches im ersten Zyklus bedarf einer deutlich höheren Energie als bei der dritten Inflation. Sowohl die Energie wie auch der zur Verformung benötigte initiale Druck zur Dehnung des Ballonschlauches sind unterschiedlich und sinken systematisch mit dem Belastungszyklus.

Dieses Verhalten erlaubt es, Rückschüsse auf die mechanische Intensität des Kathetereingriffs auf ein verformtes Gefäß zuzulassen. Insbesondere kann somit ermittelt werden, ab welchem Druck die Verformung eintritt, wie groß das verdrängte Volumen ist. Hierbei wird eine Volumen- und Druckarbeit geleistet, die in eine Verformungsenergie überführt werden kann.

Allerdings kann diese Beobachtung in der geleisteten Energie für die Katheterinflation jedoch leicht übersehen werden, wie anhand des folgenden theoretischen Auswertungsansatz ersichtlich ist.

Aus dem applizierten Druck und der Volumendifferenz lässt sich die sogenannte Volumenarbeit errechnen, die während der Inflation durch eine entsprechende Einheit (z.B. Pumpe) des Kathetersystems geleistet wird.

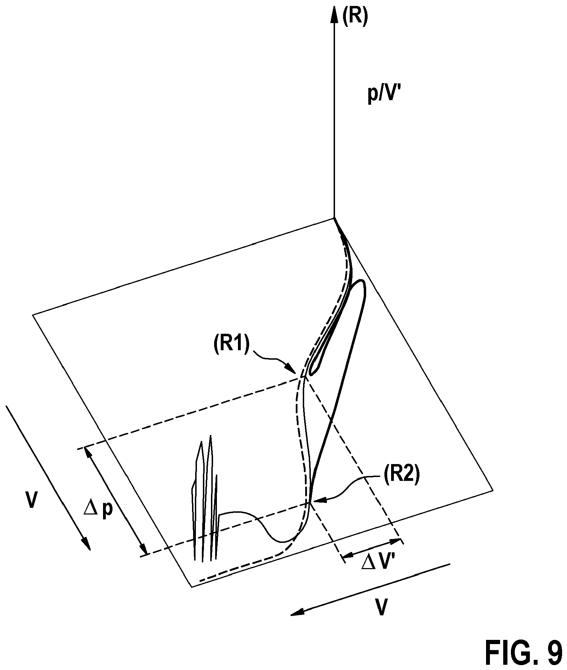

Die geleistete Volumenarbeit (schraffierter Bereich unter den jeweiligen Kurven der Figuren 3 und 4) für die in den Figuren 3 und 4 dargestellte Katheterinflation kann mittels der Formeln, die im Zusammenhang mit der Figur 9 beschrieben werden, berechnet werden.

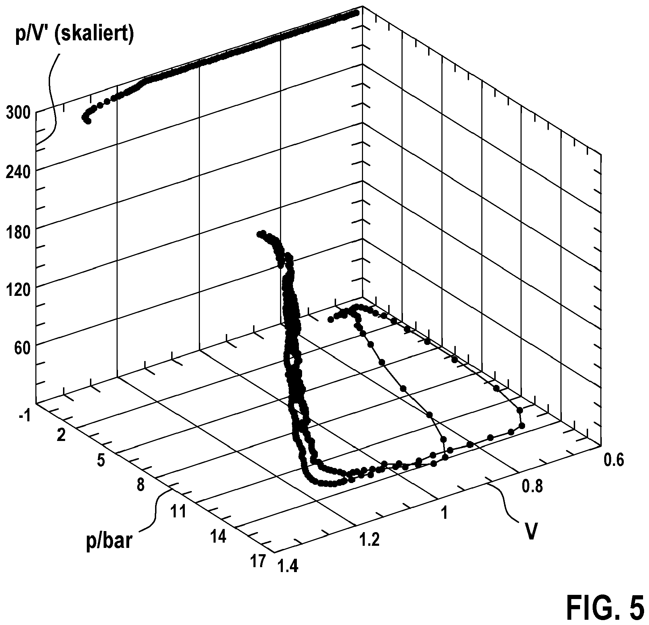

Figur 5 zeigt eine Darstellung der solchermaßen berechneten Gesamtenergie der Katheterinflation (z-Achse) entsprechend der geleisteten Volumenarbeit am Luer 24 für eine Stentinflation ohne äußere Restriktion und für eine Inflation eines Silikonschlauches.

Anhand der Grafik der Figur 5 wird deutlich, dass ohne Weiteres nur grobe Abweichungen am Luer 24 des Katheters 2 erkannt werden können. Zum Beispiel kann die Aufspannung des Silikonschlauches auf dem Ballon 22 im Gegensatz zu der Eigenspannung, die das hochfeste Ballonbauteil entwickelt, kaum erfasst werden. Die Darstellung des Füllwiderstandes des Katheters 2 kann jedoch unter Umständen empfindlich genug gewählt werden, dass auch kleine Energieaufwendungen lokalisierbar sind.

Hinsichtlich der Verformungsarbeit an einer Stenose, die regelmäßig nur einen sehr kleinen Beitrag zur aufgewandten Gesamtenergie darstellt, zeigt sich jedoch in vorteilhafter Weise, dass diese relevant und deutlich, wie auch nahezu vollständig getrennt von der Kathetercharakteristik erfassbar ist, wenn der Ballon 22 in dem Bereich einer deutlich begrenzenden Stenose noch gar nicht vollständig aufgespannt wird bzw. ist, was grundsätzlich der primären klinische Situation einer Ballondilatation entspricht.

Die Volumenarbeit wird in diesem Falle durch die verdrängte Stenose dominiert, da diese primär Einfluss auf den Volumenfluss nimmt.

In der Gesamtbetrachtung der Energie der Inflation ist jedoch der Energieverlust durch den Schaftwiderstand und die begrenzende elastische Verformung des hochfesten Ballonbauteiles dominant.

Eine verbleibende Restspannung ist dann erfassbar, wenn Sie in Konkurrenz mit der festen und gering elastischen Ballonmembran treten kann - dies ist, bis auf die Behandlung einer in-Stent-Restenose, nicht oder wenig wahrscheinlich.

In diesem Falle kann jedoch ein systematisches Vorwissen über das charakteristische Verhalten der Ballonmembran genutzt werden, um die Daten so zu präzisieren, dass auch solche Fälle als signifikante Abweichungen berechenbar sind.

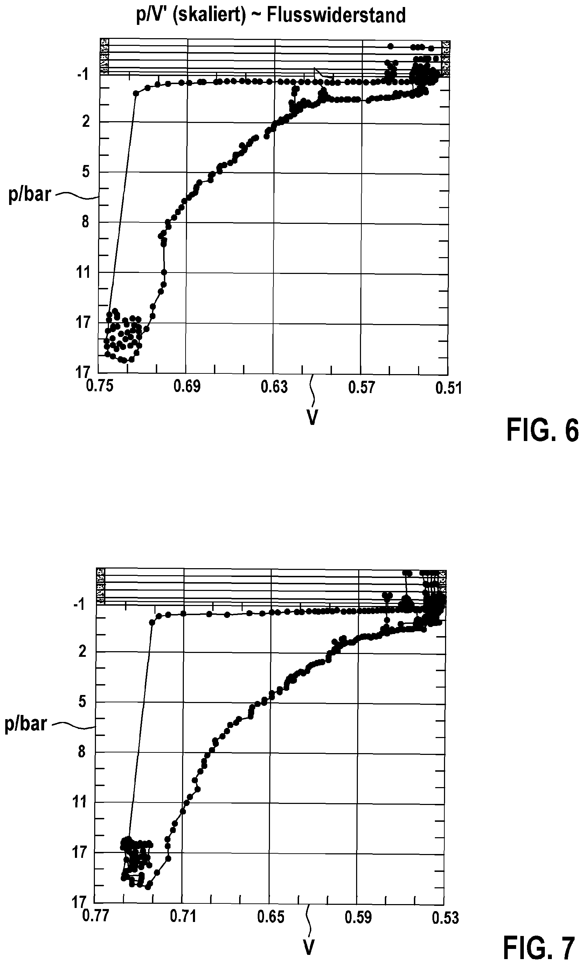

Um den Effekt einer Volumenbegrenzung im Gefäß exemplarisch zu messen, kann z.B. in den Silikonschlauch, der zuvor im Rahmen der Figuren 3 bis 5 betrachtet worden ist, noch ein Draht gelegt werden, um den Ballon 22 geometrisch einzuengen. Entsprechende Messergebnisse sind in den Figuren 6 und 7 dargestellt.

Dabei zeigt Fig. 6 die erste Inflation des Katheters 2 bzw. Ballons 22 im Silikonschlauch in Abwesenheit des Drahtes. Figur 7 zeigt die Situation mit Draht im Silikonschlauch. Beim Inflatieren des Ballons 22 drückt dieser also gegen den Draht, der damit eine Volumenbegrenzung für den Ballon 22 darstellt. Aus Fig. 6 ist ersichtlich, dass der primär exponentiellen Kinetik der Ballondeformation ein linearer Deformationsbereich (Silikonschlauch wird aufgedehnt) vorgelagert ist. Ein ähnliches primär rein elastisches Verhalten ist auch von der Präsenz von Luft im Ballon 22 zu erwarten. Figur 7 zeigt demgegenüber, dass der zusätzliche Draht primär eine geometrische Begrenzung bewirkt und dazu führt, dass die Druckzunahme relativ zum Volumen verfrüht eintritt.

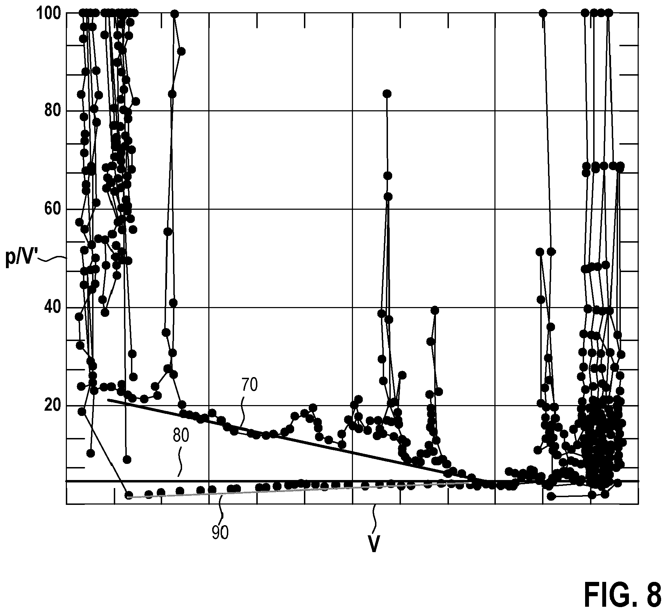

Anhand des Versuchs mit dem Silikonaußenschlauch kann auch aufgezeigt werden, wie zwischen plastischer und elastischer Verformung unterschieden werden kann. Fig. 8 zeigt diesbezüglich den Zusammenhang zwischen dem Strömungswiderstand und dem Volumen.

Die zweite Linie 80 der Fig. 8 zeigt dabei das Schaftwiderstandsniveau des Katheters. Dieser typische Schaftwiderstand wird auf identischem Niveau sowohl für die Inflation, wie auch für den Deflationszyklus ermittelt und ist am genauesten innerhalb der ersten Sekunden der Inflation (großer Fluss / hohe Druckdifferenz) zu schätzen.

Die erste Linie 70 der Fig. 8 zeigt, dass im Falle des Aufdehnens des Silikonschlauches die Kraft elastisch ansteigt, d.h., annähernd linear. Im Falle des unbelasteten Ballons 22 entsteht eine Stufe ab dem Bereich in dem die Füllung des Katheters 2 so intensiv ist, dass die Ballonmembran elastisch aufgespannt wird.

Schließlich kennzeichnet die dritte Linie 90 die Erhöhung der Flussrate / Erniedrigung des Schaftwiderstandes, die auf einer gespeicherten Energie beruht, die distal vom Ballon 22 aus den Rückfluss erhöht.

Der Schnittpunkt zwischen der dritten Linie 90 und der zweiten Linie 80 bestimmt dabei das Volumen, ab welchem nur der Deflationsdruck den Rückfluss bestimmt.

Hierbei handelt es sich um ein vom Katheterdesign abhängiges bzw. bestimmtes Volumen, das erreicht werden kann, wenn das Gefäß (oder auch ein Stent) nicht mehr in der Lage ist, einen Zusatzdruck auszuüben. Hier kann aber auch der Erfolg der Dilatation - wieviel Volumenverdrängung erfolgte plastisch - wieviel "recoil" oder Rückstellung erfolgte elastisch - abgeschätzt werden.

Aufgrund dieser Einschätzung kann eine Empfehlung an den Arzt gegeben werden, den Dilatationsvorgang ggf. zu wiederholen und eventuell auch zu welchem Durchmesser diese Dilatation erfolgen sollte.

Durch die obigen Beispiele wird deutlich, dass mittels einer zusätzlichen Sensorik (insbesondere Druck- und Volumenfluss), die Performance des Kathetersystems 1 deutlich verbessert werden kann, und zwar auf kostengünstige Weise und ohne Einschränkung der sonstigen Funktion des Kathetersystems.

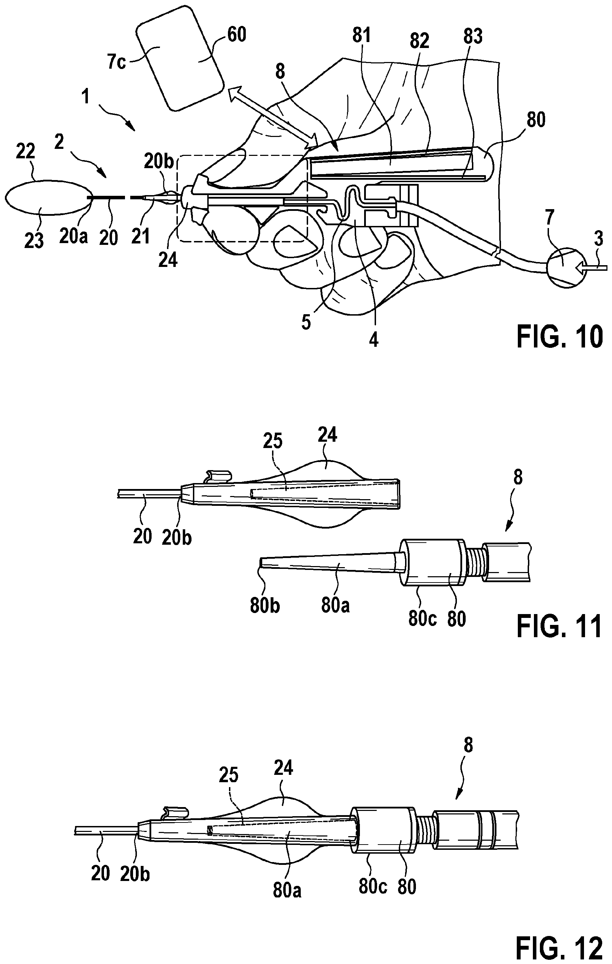

Gemäß der in der Fig. 10 gezeigten Ausführungsform weist das Ballonkathetersystem 1 diesbezüglich im Einzelnen einen Katheter 2 auf, der einen Katheterschaft 20 aufweist, der ein Lumen 21 umgibt, wobei der Katheter 2 weiterhin einen Ballon 22 aufweist, der mit einem distalen Ende 20a des Katheterschafts 20 verbunden ist, so dass zum Inflatieren des Ballons 22 ein fluides Inflationsmedium 3 in einen Balloninnenraum 23 des Ballons 22 über das Lumen 21 einleitbar ist oder zum Deflatieren des Ballons 22 aus dem Balloninnenraum 23 abziehbar ist. Weiterhin weist das Ballonkathetersystem 1 einen Drucksensor 4 auf, der dazu ausgebildet ist, einen momentanen Druck p des Inflationsmediums 3 im Lumen 21 oder im Balloninnenraum 23 beim Inflatieren oder Deflatieren des Ballons 22 zu messen, sowie einen Flusssensor 5, der dazu ausgebildet ist, einen Volumenfluss V' des Inflationsmediums 3 beim Inflatieren oder Deflatieren des Ballons 22 zu messen. Zur Auswertung dieser Messwerte bzw. Zeitserien weist das Ballonkathetersystem 1 weiterhin eine Auswertungseinheit 6 auf, die dazu konfiguriert ist, den momentanen Druck und den momentanen Volumenfluss beim Inflatieren oder Deflatieren des Ballons 22 aufzunehmen und aus diesen Werten einen zeitlichen Verlauf des Strömungswiderstands R zu ermitteln.

Das erfindungsgemäße Kathetersystem bzw. Verfahren beruht somit insbesondere auf der Erfassung des Drucks und des Volumenflusses an einem Eingang des Katheters. Der sich einstellender Strömungswiderstand setzt sich zu jedem Zeitpunkt additiv und primär dominant aus

- dem Katheterschaftwiderstand,
- den vorhandenen Ballonrestriktionen,
- dem Gewebekontakt zwischen Ballon und einer angrenzenden Gefäßwandung,
- einer Stentaufdehnung,
- und dem Widerstand, der sich letztendlich aus der Ballondimensionsbegrenzung ergibt, zusammen.

Die benötigte Leistung am Eingang bzw. Katheteranschluss 24 des Katheters 2 lässt sich berechnen zu P=dp*V',

wobei V' der Volumenstrom ist und dp die Druckdifferenz. Dies entspricht nach der Elektro-Hydraulischen Analogie der elektrischen Leistung P=U*I, wobei die Spannung U der Druckdifferenz dp und der elektrische Strom I dem Volumenstrom V' entsprechen. Die Leistung lässt sich auch mittels des Strömungswiderstands R ausdrücken, der in der Analogie dem Ohmschen Widerstand entspricht: $P=V'^2*R$.

Die Energie, die in den Katheter gesteckt wird, ergibt sich durch Multiplikation der Leistung mit der Zeit (wobei V'*t das Volumen V ergibt): $E=dp*V$

Interessant ist hierbei, dass die momentane Leistung, aber auch die entsprechende Leistungsänderungen, in dem zeitlichen Verlauf der Gesamtenergie über die Kenntnis des Volumenflusses und des Strömungswiderstands - oder auch einem Widerstandsbeitrag - zu einem bestimmten Zeitpunkt ermittelbar sind.

Das bedeutet, dass der Widerstandszuwachs (z.B. durch die Restriktion eines Silikonschlauches) über die relative Widerstandsänderung getrennt von dem Gesamtsystem betrachtet werden kann (sozusagen interpretiert als eine elektrische Reihenschaltung).

Weiterhin ist der zeitlich simultane Volumenfluss bekannt. Dadurch wird der Druck, den der Ballon ausübt um, die Stenose zu öffnen, indirekt ermittelbar, wie auch die Energie die hierfür umgesetzt wurde.

Da die Energie, die notwendig ist, um den Katheter zu füllen dominant ist, handelt es sich bei diesem Effekt nur um eine zeitlich begrenzte und geringfügige Abweichung von dem typischen unbelasteten Katheterverhalten. Verbleibende Restspannungen werden kaum schätzbar, da folgend die hochfeste Ballonmembran dominant für die Volumenarbeit wird.

Aus diesem Grunde kann die Kenntnis eines systematischen Inflationsverhalten dazu beitragen eine Präzisierung dieser Beobachtungen zu ermöglichen, sie ist aber solange die Ballonmembran noch nicht elastisch ausgespannt wird bzw. begrenzend wirkt im Prinzip nicht zwingend notwendig.

Gemäß Figur 9 wird zur Auswertung der Messdaten vorzugsweise über das Produkt aus Druck und Strömungswiderstand zwischen einem ersten Punkt R1 und einem zweiten Punkt R2 integriert, beziehungsweise werden diese Produkte für die jeweiligen einzelnen Messwerte aufsummiert, um die gesamte Energie zu bestimmen, die zur Inflation des Ballons zwischen Ri, zu dem der Ballon beim Inflatieren eine Gefäßwand des Patienten kontaktiert, und R2, zu dem die Stenose dilatiert ist, verrichtet wurde:

$$W_{gesamt} = \int_{t_1}^{t_2} \frac{p^2(t)}{V'(t)} \, t \, dt$$

mit Druck p, Volumenstrom V', der Zeit ti des Punktes R1 und der Zeit $t_2$ des Punktes R2.

Mit dem Strömungswiderstand $R = \frac{p}{V'}$ ergibt sich:

$$W_{gesamt} = \int_{t_1}^{t_2} p(t)R(t) \, dt$$

Der Strömungswiderstand R(t) setzt sich hierbei aus einem Strömungswiderstand $R_{Stenose}$ resultierend aus der Dilation der Stenose und aus einem Strömungswiderstand $R_{Ballon}$ resultierend aus der Dehnung des Ballons zusammen. Damit kann die Energie $W_{Stenose}$, die für die Dilation der Stenose des Patienten notwendig war, wie folgt berechnet werden:

$$W_{Stenose} = \int_{t_1}^{t_2} p(t)(R(t) - R_{Ballon}(t)) \, dt$$

Der zeitliche Verlauf des Strömungswiderstands R wird von der Auswertungseinheit ermittelt. Der zeitliche Verlauf des Strömungswiderstands $R_{Ballon}$ zwischen den Zeitpunkten $t_1$ und $t_2$ wird von der Auswertungseinheit interpoliert:

$$R_{Ballon}(t) = \frac{(R_{Ballon}(t_2) - R_{Ballon}(t_1)) * (t - t_1)}{t_2 - t_1} + R_{Ballon}(t_1)$$

Hieraus lässt sich die Energie $W_{Stenose}$, die für die Dilation der Stenose des Patienten notwendig war analytisch, numerisch oder durch Summierung der Produkte $p(t)(R(t)-R_{Ballon}(t))$ für die einzelnen Messwerte in dem Zeitintervall $[t_1; t_2]$ bestimmen.

Hieraus lässt sich als interessante Größe sowohl der relative Druck, der zu Verformung überschritten werden musste, wie auch die Verformungsenergie für diesen "Teilprozess" berechnen. Weitere interessante Größen, die nach dieser Auswertungsmethode ermittelt werden können, sind z.B.

- einen Zeitpunkt zu dem der Ballon beim Inflatieren eine Gefäßwand des Patienten kontaktiert, oder
- einen Zeitpunkt zu dem der Ballon beim Deflatieren einen Gefäßkontakt wieder verliert.

Wie weiterhin aus Fig. 10 ersichtlich ist, kann das Ballonkathetersystem 1 eine Pumpe 7 zum Inflatieren und/oder Deflatieren des Ballons 22 mit dem Inflationsmedium 3 aufweisen.

Gemäß einer Ausführungsform kann hierbei vorgesehen sein, dass der Drucksensor in die Pumpe 7 integriert ist. Der besagte Flusssensor des Ballonkathetersystems 1 könnte dann in eine Sensoreinheit integriert sein, die dazu ausgebildet ist, die Pumpe 7 mit dem Katheteranschluss 24 zu verbinden.

Vorzugsweise ist jedoch gemäß Fig. 10 vorgesehen, dass sowohl der Drucksensor 4 als auch der Flusssensor 5 in die Sensoreinheit 8 integriert sind, die dazu ausgebildet ist, den Katheteranschluss 24 und die Pumpe 7 strömungstechnisch miteinander zu verbinden (d.h. das fluide Inflationsmedium wird über die Sensoreinheit 8 geführt.

Die Sensoreinheit 8 kann gemäß Fig. 10 dazu ausgebildet sein, den gemessenen momentanen Druck p und den gemessenen momentanen Volumenfluss V' über eine Funkverbindung bzw. drahtlos an die Auswertungseinheit 6 zu übermitteln. Die Sensoreinheit 8 kann hierzu eine Spule 82 aufweisen (insbesondere zur Nahfeldkommunikation), wobei die Spule 82 auch zum Laden eines Energiespeichers 81 dienen kann. Weiterhin kann die Sensoreinheit 8 eine Antenne 83 (z.B. 433 MHz) zum Senden und Empfangen von Funksignalen an/von der Auswertungseinheit aufweisen.

Weiterhin weist das Ballonkathetersystem 1 vorzugsweise eine Datenerfassungseinheit 60 aufweist, die zur Eingabe einer oder mehrere den Katheter 2 charakterisierenden Parameter konfiguriert ist, um insbesondere eine Identifikation des verwendeten Katheters 2 zu ermöglichen, wobei die Auswertungseinheit 6 dazu konfiguriert, ist den jeweiligen Parameter bei der Bestimmung einer zu ermittelnden Größe (siehe oben) zu verwenden, um die Signalauswertung und Datenaufbereitung zu präzisieren.

Der Katheteranschluss 24 weist weiterhin in der Regel ein gewisses Eingangslumen 25 aufweist, das mit dem Lumen 21 des Katheterschafts 20 verbunden ist bzw. in Strömungsverbindung steht (vgl. Figuren 10 bis 12). Da Luft, die sich in diesem Bereich sammeln kann hinsichtlich der Auswertung der Messdaten eine Störgröße darstellen, ist vorzugsweise vorgesehen, dass die Sensoreinheit 8 einen z.B. konisch zulaufenden Auslassbereich 80a zum Auslassen des Inflationsmediums 3 aus der Sensoreinheit 8 aufweist, wobei der Auslassbereich 80a dazu ausgebildet ist, formschlüssig in das Eingangslumen 25 einzugreifen.

Der Auslassbereich 80a kann z.B. durch einen konisch zulaufenden Gehäuseabschnitt 80a eines Gehäuses 80 der Sensoreinheit 8 gebildet sein, wobei der Gehäuseabschnitt 80a an einem Ende einen Auslass 80b zum Auslassen des Inflationsmediums 3 aufweist. Das Gehäuse 80 dient zur Aufnahme des Drucksensors 4 und des Flusssensors 5 sowie insbesondere der oben beschriebenen weiteren Komponenten der Sensoreinheit. Wenn der Katheteranschluss 24 als Luer-Verbinder ausgebildet ist, ist am Gehäuse 80 ein entsprechender Verbindungsbereich 80c vorgesehen, der mit dem Luer-Verbinder 24 in Eingriff treten kann, um den Katheteranschluss 24 und das Gehäuse 80 bzw. die Sensoreinheit 8 miteinander zu verbinden (nach einem Luer-Prinzip).

Der Katheteranschluss 24 (z. B. Luer-Verbinder) und der Auslassbereich 80a sind damit so gestaltet, dass im Katheteranschluss 24 bzw. Eingangslumen 25 möglichst wenig Luft bzw. Restvolumen verbleibt, so dass bei der Auswertung nicht zwischen Luft im Katheteranschluss 24 und Luft im Ballon 22 unterschieden werden muss. Wird Luft im Katheteranschluss 24 ausgeschlossen, kann durch Modellbindung die Luftmenge im Ballon geschätzt werden (da Sie ein rein lineares Kompressionsverhalten aufweist).

Alternativ zu dem wie vorliegend ausgestalteten Katheteranschluss/Auslassbereich kann das Restvolumen im Katheteranschluss rechnerisch korrigiert werden, wenn das Volumen der Luft im Katheter durch entsprechende Vorcharakterisierung bekannt ist.

Die Sensoreinheit 8 stellt mit Vorteil ein kompaktes Bindungsglied zwischen Pumpe 7 und Katheteranschluss 24 dar und bildet einen Strömungspad für das Inflationsmedium 3, der vorzugsweise die gesamte Sensorik, d.h., Drucksensor 4 und Flusssensor 5, aufweist. Hierbei erweist es sich von Vorteil, den Flusssensor 5 als Coriolis-Flusssensor auszugestalten, da dieser leicht sterilisierbar ist sowie eine Strömungsumkehr des Inflationsmediums 3 erlaubt.

Aufgrund der Erfindung kann der Arzt bei einer Angioplastie mit Vorteil zusätzliche Informationen erhalten, die die Durchführung der Angioplastie sowie das Ergebnis des Eingriffs verbessern. Insbesondere wirkt bei der Inflation des Ballons der Gewebekontakt begrenzend, wobei das Volumen und der Druck zum Zeitpunkt des Gewebekontaktes ermittelbar sind.

Anhand der Messdaten können mit Vorteil weiterhin die folgende Parameter bestimmt werden:

- ein aktueller mittlerer Gefäßdurchmesser,
- ein effektiver Gefäß-Gegendruck,
- ein aktuelles Volumendefizit der Dilatation,
- ein Dilatationsdruck, ab dem das Gewebe beginnt nachzugeben,
- eine für den Dilatationsvorgang erforderliche Energie.

Weiterhin ermöglicht die Erfindung mit Vorteil folgende retrospektive Angaben zur Dilatation:

- erzwungener mittlerer Durchmesser des Gefäßes
- elastischer / plastischer Volumenanteil der Dilatation
- die Definition eines Formfaktordefizites.

Hieraus kann der behandelnde Arzt weiterhin abschätzen, ob ggf. eine Nachdilatation empfehlenswert ist.

**Patentansprüche**

1. Ballonkathetersystem (1), mit:

   - einem Katheter (2), der einen Katheterschaft (20) aufweist, der ein Lumen (21) umgibt, wobei der Katheter (2) weiterhin einen Ballon (22) aufweist, der mit einem distalen Ende (20a) des Katheterschafts (20) verbunden ist, so dass zum Inflatieren des Ballons (22) ein fluides Inflationsmedium (3) in einen Balloninnenraum (23) des Ballons (22) über das Lumen (21) einleitbar ist oder zum Deflatieren des Ballons (22) aus dem Balloninnenraum (23) abziehbar ist,

   **dadurch gekennzeichnet,**
   **dass** das Ballonkathetersystem (1) einen Drucksensor (4) aufweist, der dazu ausgebildet ist, einen momentanen Druck (p) des Inflationsmediums (3) im Lumen (21) beim Inflatieren oder Deflatieren des Ballons (22) zu messen, sowie einen Flusssensor (5), der dazu ausgebildet ist, einen Volumenfluss (v) des Inflationsmediums (3) beim Inflatieren oder Deflatieren des Ballons (22) zu messen, und wobei das Ballonkathetersystem (1) eine Auswertungseinheit (6) aufweist, die dazu konfiguriert ist, den momentanen Druck und den momentanen Volumenfluss beim Inflatieren oder Deflatieren des Ballons (22) aufzuzeichnen und hieraus einen zeitlichen Verlauf des Strömungswiderstands (R) zu ermitteln.

2. Ballonkathetersystem nach Anspruch 1, **dadurch gekennzeichnet, dass** die Auswertungseinheit dazu konfiguriert ist, mit Hilfe des Strömungswiderstands (R) zumindest eine Größe der folgenden Größen zu bestimmen:

   - einen Zeitpunkt zu dem der Ballon beim Inflatieren eine Gefäßwand des Patienten kontaktiert,
   - eine Energie, die für eine Dilation einer Stenose des Patienten notwendig war,
   - einen Zeitpunkt zu dem die Stenose dilatiert ist.

3. Ballonkathetersystem nach einem der vorhergehenden Ansprüche, **dadurch gekennzeichnet, dass** der Katheter (2) einen mit dem Lumen (21) verbundenen Katheteranschluss (24) aufweist, wobei der Drucksensor (4) dazu ausgebildet ist, den momentanen Druck am Katheteranschluss (24) zu messen und/oder wobei der Flusssensor (5) dazu ausgebildet ist, den Volumenfluss am Katheteranschluss (24) zu messen.

4. Ballonkathetersystem nach einem der vorhergehenden Ansprüche, **dadurch gekennzeichnet, dass** der Flusssensor (5) ein Coriolis-Flusssensor ist.

5. Ballonkathetersystem, **dadurch gekennzeichnet, dass** das Ballonkathetersystem eine Pumpe (7) zum Inflatieren und/oder Deflatieren des Ballons (22) mit dem Inflationsmedium (3) aufweist.

6. Ballonkathetersystem nach Anspruch 5, **dadurch gekennzeichnet, dass** der Drucksensor (4) in die Pumpe (7) integriert ist.

7. Ballonkathetersystem nach Anspruch 3 oder einem der Ansprüche 4 bis 6 soweit rückbezogen auf Anspruch 4, **dadurch gekennzeichnet, dass** der Flusssensor (5) des Ballonkathetersystems (1) in eine Sensoreinheit (8) integriert ist, die dazu ausgebildet ist, die Pumpe (7) mit dem Katheteranschluss (24) zu verbinden.

8. Ballonkathetersystem nach den Ansprüchen 3 und 5, **dadurch gekennzeichnet, dass** der Drucksensor (4) und der Flusssensor (5) in eine Sensoreinheit (8) integriert sind, die dazu ausgebildet ist, den Katheteranschluss (24) und die Pumpe (7) miteinander zu verbinden

9. Ballonkathetersystem nach Anspruch 8, **dadurch gekennzeichnet, dass** die Sensoreinheit (8) dazu ausgebildet ist, den gemessenen momentanen Druck und den gemessenen momentanen Volumenfluss über eine Funkverbindung an die Auswertungseinheit (6) zu übermitteln.

10. Ballonkathetersystem nach einem der vorhergehenden Ansprüche, **dadurch gekennzeichnet, dass** das Ballonkathetersystem (1) eine Datenerfassungseinheit (60) aufweist, die zur Eingabe einer oder mehrere den Katheter (2) charakterisierenden Parameter konfiguriert ist.

11. Ballonkathetersystem nach den Ansprüchen 2 und 10, **dadurch gekennzeichnet, dass** die Auswertungseinheit (6) dazu konfiguriert, ist den jeweiligen Parameter bei der Bestimmung der mindestens einen Größe zu verwenden.

12. Ballonkathetersystem nach Anspruch 3 und nach einem der Ansprüche 7 bis 9, **dadurch gekennzeichnet, dass** der Katheteranschluss (24) ein Eingangslumen (25) aufweist, das mit dem Lumen (21) des Katheterschafts (20) verbunden ist, und wobei die Sensoreinheit (8) einen Auslassbereich (80a) zum Auslassen des Inflationsmediums (3) aus der Sensoreinheit (8) aufweist, wobei der Auslassbereich (80a) dazu ausgebildet ist formschlüssig in das Eingangslumen (25) einzugreifen.

13. Verfahren zum Inflatieren eines Ballons eines Katheters mit einem fluiden Inflationsmedium, aufweisend die Schritte:

   - Messen und Aufzeichnen eines momentanen Volumenflusses (v) des Inflationsmediums (3) und eines momentanen Drucks (p) des Inflationsmediums (3) beim Inflatieren oder Deflatieren des Ballons (22), und
   - automatisches Bestimmen eines zeitlichen Verlaufs des Strömungswiderstands (R) beim Inflatieren oder Deflatieren des Ballons (22) mittels gemessener Werte des momentanen Drucks und des momentanen Volumenflusses.

14. Verfahren nach Anspruch 13, weiterhin aufweisend den Schritt: Automatisches Bestimmen zumindest einer Größe der folgenden Größen mit Hilfe des Strömungswiderstands (R):

   - einen Zeitpunkt, zu dem der Ballon (22) beim Inflatieren eine Gefäßwand des Patienten kontaktiert,
   - eine Energie, die für eine Dilation einer Stenose des Patienten notwendig war,
   - ein Zeitpunkt, zu dem die Stenose dilatiert ist.

FIG. 1

FIG. 2

p/V' (skaliert)

100

80

60

40

20

-1

V

0.51

0.57

0.63

0.69

0.75

2   5   8   11   14   17

p/bar

**FIG. 3**

p/V' (skaliert)

100

80

60

40

20

-1

V

0.5

0.56

0.62

0.68

0.74

2   5   8   11   14   17

p/bar

**FIG. 4**

FIG. 5

p/V' (skaliert) ~ Flusswiderstand

FIG. 6

p/V' (skaliert) ~ Flusswiderstand

FIG. 7

FIG. 8

FIG. 9

**FIG. 10**

**FIG. 11**

**FIG. 12**

**Europäisches Patentamt**
**European Patent Office**
**Office européen des brevets**

## EUROPÄISCHER RECHERCHENBERICHT

Nummer der Anmeldung

EP 22 21 4795

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2010/113939 A1 (MASHIMO HIROSHI [US] ET AL) 6. Mai 2010 (2010-05-06) * Absätze [0013], [0023] – [0025], [0027] – [0028], [0031], [0038] – [0040]; Abbildungen 1-3B * ----- | 1-4,7-14 | INV. A61M25/10 |
| A | US 2014/276198 A1 (DUNUNG RONAK [US] ET AL) 18. September 2014 (2014-09-18) * Absätze [0027] – [0033], [0052]; Abbildung 3 * ----- | 1-4,7-14 | |
| A | EP 1 931 272 B1 (COVIDIEN LP [US]) 12. November 2014 (2014-11-12) * Absatz [0085]; Abbildung 17 * ----- | 1-4,7-14 | |
| A | EP 2 579 765 B1 (OMEQ MEDICAL LTD [IL]) 7. August 2019 (2019-08-07) * Absätze [0052] – [0053]; Anspruch 4; Abbildung 3 * ----- | 1-4,7-14 | |
| A | WO 2014/097301 A1 (OMEQ MEDICAL LTD [IL]) 26. Juni 2014 (2014-06-26) * Absätze [0054] – [0058]; Abbildung 2 * ----- | 1-4,7-14 | RECHERCHIERTE SACHGEBIETE (IPC) A61M A61B |
| A | US 2022/287730 A1 (CHISENA ROBERT [US] ET AL) 15. September 2022 (2022-09-15) * Absatz [0059] * ----- | 1-4,7-14 | |
| A | US 2011/137580 A1 (BARTELS FRANK [DE] ET AL) 9. Juni 2011 (2011-06-09) * Absätze [0039] – [0040] * ----- | 4 | |
| A | CN 107 050 627 A (HUZHOU NO 1 PEOPLE'S HOSPITAL) 18. August 2017 (2017-08-18) * siehe Übersetzung; Abbildung 1 * ----- | 1-4,7-14 | |

~~Der vorliegende Recherchenbericht wurde für alle Patentansprüche erstellt~~

| Recherchenort | Abschlußdatum der Recherche | Prüfer |
|---|---|---|
| München | 9. Juni 2023 | Berndorfer, Urs |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches
Patentamt

European
Patent Office

Office européen
des brevets

Nummer der Anmeldung

EP 22 21 4795

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

**Siehe Ergänzungsblatt B**

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

**1-4, 13, 14(vollständig); 7-12(teilweise)**

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

**MANGELNDE EINHEITLICHKEIT
DER ERFINDUNG
ERGÄNZUNGSBLATT B**

**Nummer der Anmeldung**

**EP 22 21 4795**

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-4, 13, 14 (vollständig); 7-12 (teilweise)

    Ballonkathetersystem mit einem Katheter, der einen Katheterschaft aufweist, der ein Lumen umgibt, wobei der Katheter weiterhin einen Ballon aufweist, der mit einem distalen Ende des Katheterschafts verbunden ist, sowie mit Sensorik.

    ———

2. Ansprüche: 5, 6 (vollständig); 7-12 (teilweise)

    Ballonkathetersystem mit einem Katheter, der einen Katheterschaft aufweist, der ein Lumen umgibt, wobei der Katheter weiterhin einen Ballon aufweist, der mit einem distalen Ende des Katheterschafts verbunden ist, sowie mit Pumpe.

    ———

**ANHANG ZUM EUROPÄISCHEN RECHERCHENBERICHT
ÜBER DIE EUROPÄISCHE PATENTANMELDUNG NR.**

EP 22 21 4795

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09−06−2023

| Im Recherchenbericht angeführtes Patentdokument | Datum der Veröffentlichung | Mitglied(er) der Patentfamilie | Datum der Veröffentlichung |
|---|---|---|---|
| US 2010113939 A1 | 06−05−2010 | US 2010113939 A1 | 06−05−2010 |
| | | WO 2008042347 A2 | 10−04−2008 |
| US 2014276198 A1 | 18−09−2014 | EP 2967372 A1 | 20−01−2016 |
| | | US 2014276198 A1 | 18−09−2014 |
| | | WO 2014149708 A1 | 25−09−2014 |
| EP 1931272 B1 | 12−11−2014 | AU 2006302688 A1 | 19−04−2007 |
| | | BR PI0616840 A2 | 05−07−2011 |
| | | CA 2624792 A1 | 19−04−2007 |
| | | EP 1931272 A2 | 18−06−2008 |
| | | EP 2604212 A1 | 19−06−2013 |
| | | EP 2604213 A1 | 19−06−2013 |
| | | EP 2799027 A1 | 05−11−2014 |
| | | JP 5222144 B2 | 26−06−2013 |
| | | JP 5583734 B2 | 03−09−2014 |
| | | JP 2009509713 A | 12−03−2009 |
| | | JP 2013066725 A | 18−04−2013 |
| | | US 2006095032 A1 | 04−05−2006 |
| | | US 2010234840 A1 | 16−09−2010 |
| | | US 2012004654 A1 | 05−01−2012 |
| | | US 2013131661 A1 | 23−05−2013 |
| | | US 2015038958 A1 | 05−02−2015 |
| | | WO 2007044244 A2 | 19−04−2007 |
| EP 2579765 B1 | 07−08−2019 | CN 103281950 A | 04−09−2013 |
| | | EP 2579765 A2 | 17−04−2013 |
| | | US 2013085413 A1 | 04−04−2013 |
| | | WO 2011158227 A2 | 22−12−2011 |
| WO 2014097301 A1 | 26−06−2014 | CN 104994778 A | 21−10−2015 |
| | | EP 2934302 A1 | 28−10−2015 |
| | | US 2015342635 A1 | 03−12−2015 |
| | | WO 2014097301 A1 | 26−06−2014 |
| US 2022287730 A1 | 15−09−2022 | AU 2022217158 A1 | 29−06−2023 |
| | | US 2022287730 A1 | 15−09−2022 |
| | | WO 2022169778 A1 | 11−08−2022 |
| US 2011137580 A1 | 09−06−2011 | DE 102006062552 A1 | 03−07−2008 |
| | | TW 200900665 A | 01−01−2009 |
| | | US 2011137580 A1 | 09−06−2011 |
| | | WO 2008080552 A2 | 10−07−2008 |
| CN 107050627 A | 18−08−2017 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014210450 A